# EUROPEAN PATENT SPECIFICATION

(11) **EP 4 389 298 B1**
(45) Date of publication and mention of the grant of the patent: **13.08.2025**
(21) Application number: 23218694.0
(22) Date of filing: 20.12.2023
(51) Int. Cl.: B05B 12/20

(54) **FIXTURES FOR MASKING METAL PARTS**
BEFESTIGUNGEN ZUR MASKIERUNG VON METALLTEILEN
FIXATIONS POUR MASQUER DES PIÈCES MÉTALLIQUES

(30) Priority: 22.12.2022 US 202263476673 P
(43) Date of publication of application: 26.06.2024
(73) Proprietor: Howmedica Osteonics Corp., Mahwah, NJ 07430 (US)
(72) Inventor: CROUS, Heinrich George, CARRIGTWOHILL, T45 RF20 (IE)
(74) Representative: Regimbeau

(56) References cited:
- WO-A1-2021/090160
- US-A1- 2009 082 866
- US-A1- 2009 254 191
- US-A1- 2018 250 019

## Description

### BACKGROUND OF THE INVENTION

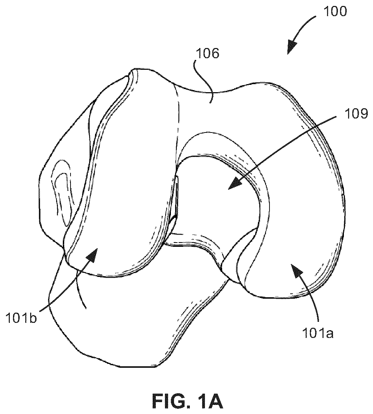

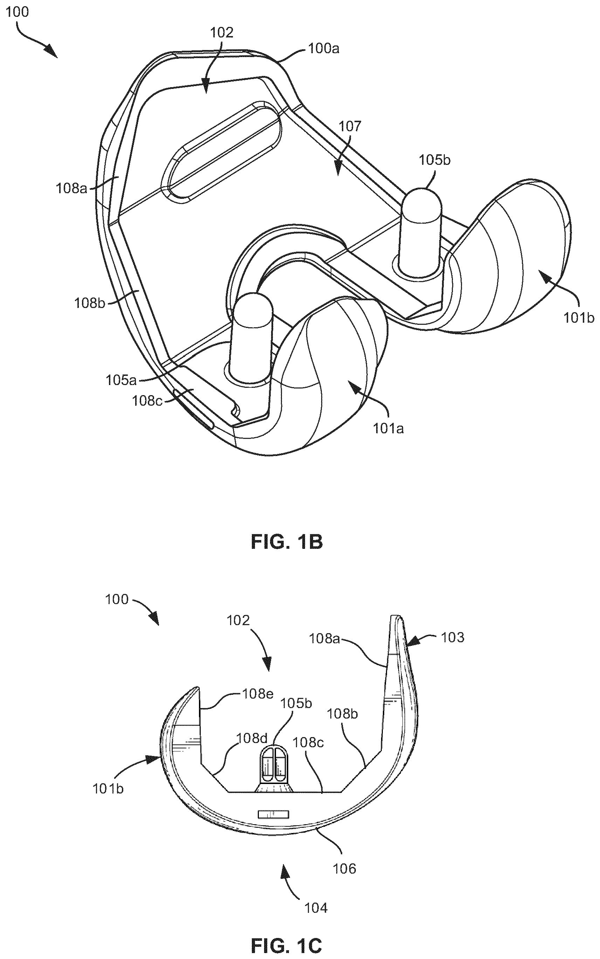

Orthopedic implants used in joint replacement often have an articular side that articulates with another orthopedic joint implant and a fixation side that is intended to be secured to bone and/or another implant, such as an intramedullary stem. For example, a femoral component 100 of a total knee arthroplasty ("TKA") is shown in FIGs. 1A-1C and depicts a common configuration for a "five-cut" femur. In this regard, femoral component 100 has a fixation side (i.e., bone facing side) 102 and an articular side 104. Articular side 104 generally includes first and second condyles 101a-b separated by an intercondylar notch 109 and an anterior flange 103 that together define an articular surface 106 of femoral component 100. Fixation side 102 generally includes five intersecting planar surfaces 108a-e, namely an anterior surface 108a, anterior chamfer surface 108b, distal surface 108c, posterior chamfer surface 108d, and posterior surface 108e. Each of these surfaces 108a-e are adapted to contact or interface with a corresponding resection of a distal femur. Additionally, a pair of pegs 105a-b extend from distal surface 108c for receipt in corresponding bone openings of a femur.

Thus, articular side 104 is configured to articulate with another joint component (i.e., polymer insert of a tibial component), and bone contacting side 102 is configured to be fixed or otherwise secured to a bone (i.e., distal femur). In this regard, each side 102, 104 may be optimized for their particular functions-articulation and fixation. For example, fixation side 102, such as surfaces 108a-e and pegs 105a-b, may be provided with a porous structure 107 for bony ingrowth, and articular side 104 can be coated for wear resistance. It has been found that applying a coating, such as a titanium nitride ("TiN"), to articular surfaces of a metallic implant, such as femoral component 100, can harden the articular interface and reduce friction thereby improving the implant's wear profile. However, coatings of this nature, which may be applied via physical vapor deposition ("PVD"), typically also expose fixation side 102 to the coating material which may negatively impact its fixation function. The closest prior art is document WO 2021/090160 A1, which discloses a method of surface coating a femoral component, the femoral component comprising an articular side, a bone facing side, and a peripheral edge defining an intersection between the articular side and bone facing side, the bone facing side having a first planar surface having a first perimeter and a second planar surface having a second perimeter, the perimeters of the first and second planar surfaces defining at least a portion of the peripheral edge of the femoral component. Thus, it may be desirable to shield fixation side 102 during the coating process so that it remains free of the coating material. However, properly shielding a fixation side of an implant has proven difficult particularly in mass production of orthopedic implants. Therefore, further improvements are desirable.

### BRIEF SUMMARY OF THE INVENTION

The invention is defined in claim 1. According to an aspect of the present disclosure, a fixture for masking a femoral component during a surface coating process is disclosed herein. The femoral component includes an articular side, a bone facing side, and a peripheral edge that defines an intersection between the articular side and bone facing side. The bone facing side has a first planar surface that has a first perimeter and a second planar surface that has a second perimeter. The perimeters of the first and second planar surfaces define at least a portion of the peripheral edge of the femoral component.

The fixture includes a first panel and a second panel. The first panel has a first interface surface that defines a first perimeter of the first panel. The first perimeter of the first panel has substantially the same shape as the first perimeter of the first planar surface of the femoral component. The second panel is connected to the first panel and has a second interface surface that defines a second perimeter of the second panel. The second perimeter of the second panel has substantially the same shape as the second perimeter of the second planar surface of the femoral component. When the fixture is mounted to the bone facing side of the femoral component, the first interface surface engages the first planar surface of the femoral component and covers an entirety thereof, and the second interface surface engages the second planar surface of the femoral component and covers an entirety thereof.

Additionally, the first panel may include a first sidewall that extends downwardly from the first perimeter, and the second panel may include a second sidewall that extends downwardly from the second perimeter. The first and second sidewalls may overlap at least a portion of the peripheral edge of the femoral component when the fixture is mounted to the bone facing side of the femoral component. The first and second sidewalls may be perpendicular to the first and second interface surfaces of the first and second panels, respectively. Further, the first and second sidewalls may intersect the first and second interface surfaces, respectively, at an oblique angle. The oblique angle may be 45 degrees. Furthermore, the first and second sidewalls of the respective first and second panels may be each offset from the peripheral edge of the femoral component by a distance. The distance may be 0.5 mm.

Continuing with this aspect, the first and second panels may be rigidly fixed to each other. Alternatively, the first and second panels may be pivotable relative to each other via a hinge. The hinge may be a flexible hinge. Additionally, the flexible hinge and first and second panels may be integrally connected so as to form a monolithic structure.

Also, the fixture may further include a third panel connected to the second panel. The third panel may include a third interface surface that defines a third perimeter of the third panel. The third perimeter may have substantially the same shape as a third perimeter of a third planar surface of the femoral component. The third panel may also include first and second sheaths that extend from a mask side of the fixture and first and second sheath openings that extend into the respective first and second sheaths from an implant facing side of the fixture. The first and second sheath openings may be configured to respectively receive first and second fixation members extending from the third planar surface of the femoral component when the fixture is mounted to the bone facing side of the femoral component.

Additionally, the fixture may include a fourth and fifth panel that have respective fourth and fifth interface surfaces corresponding to respective fourth and fifth planar surfaces of the femoral component. Further, a recess may intersect the third, fourth, and fifth panels of the fixture so as to define first and second condylar portions of the fixture. The first and second panels may define an anterior flange portion of the fixture. Furthermore, the fixture may include a first tab extending from the first panel, and a second tab extending from the fifth panel. The second tab may extend across the recess.

In yet another embodiment of present disclosure, the first and second perimeters of the first and second panels may be flush with the first and second perimeters of the first and second planar surfaces of the femoral component when the fixture is mounted to the bone facing side of the femoral component.

In yet another aspect of the present disclosure, an assembly prepared for a surface coating process includes a femoral component and a fixture. The femoral component has an articular side, a bone facing side, and a peripheral edge that defines an intersection between the articular side and bone facing side. The bone facing side has a first planar surface having a first perimeter and a second planar surface that has a second perimeter The perimeters of the first and second planar surfaces define at least a portion of a peripheral edge of the femoral component.

The fixture includes a first panel and a second panel. The first panel has a first interface surface that defines a first perimeter of the first panel. The first perimeter of the first panel has substantially the same shape as the first perimeter of the first planar surface of the femoral component. The second panel is connected to the first panel and has a second interface surface that defines a second perimeter of the second panel. The second perimeter of the second panel has substantially the same shape as the second perimeter of the second planar surface of the femoral component. When the fixture is mounted to the bone facing side of the femoral component, the first interface surface engages the first planar surface of the femoral component and covers an entirety thereof, and the second interface surface engages the second planar surface of the femoral component and covers an entirety thereof.

### BRIEF DESCRIPTION OF THE DRAWINGS

The features, aspects, and advantages of the present invention will become better understood with regard to the following description, appended claims, and accompanying drawings in which:
FIG. 1A is a perspective view of a femoral component according to an embodiment of the present disclosure connected to a distal femur.
FIG. 1B is a top perspective view of the femoral component of FIG. 1A.
FIG. 1C is a side elevational view of the femoral component of FIG. 1A.
FIG. 2A is a perspective view of a fixture according to an embodiment of the present disclosure.
FIG. 2B is a side elevational view of the fixture of FIG. 2A.
FIG. 2C is a top view of the fixture of FIG. 1A.
FIG. 2D is a bottom view of the fixture of FIG. 1A.
FIG. 2E is a rear elevational view of the fixture of FIG. 1A.
FIG. 2F is a front elevational view of the fixture of FIG. 1A.
FIG. 2G is a bottom perspective view of the fixture of FIG. 1A.
FIG. 2H is another bottom perspective view of the fixture of FIG. 1A.
FIG. 3A is a top perspective view of an assembly comprised of the fixture of FIG. 2A coupled to the femoral component of FIG 1A.
FIG. 3B is a rear elevational view of the assembly of FIG. 3A.
FIG. 3C is a side elevational view of the assembly of FIG. 3A.
FIG. 3D is a cross-sectional view of the assembly of FIG. 3A taken along the line D-D of FIG. 3B.
FIG. 4A is a perspective view of a fixture according to another embodiment of the present disclosure.
FIG. 4B is a perspective view of the fixture of FIG. 4A coupled to a femoral component.
FIG. 5 is a perspective view of a fixture according to a further embodiment of the present disclosure coupled to a femoral component.
FIG. 6 is a perspective view of a fixture according to an even further embodiment of the present disclosure coupled to a femoral component.
FIG. 7 is a perspective view of a fixture according to yet another embodiment of the present disclosure coupled to a femoral component.

### DETAILED DESCRIPTION

As used herein, the term "proximal," when used in connection with a surgical tool or device, or components of a device, refers to the end of the device closer to the user of the device when the device is being used as intended. On the other hand, the term "distal," when used in connection with a surgical tool or device, or components of a device, refers to the end of the device farther away from the user when the device is being used as intended. However, when used in connection with the human body, the term "proximal" means closer to the heart, and the term "distal" means further from the heart. As used herein, the terms "substantially," "generally," "approximately," and "about" are intended to mean that slight deviations from absolute are included within the scope of the term so modified, such as deviations of up to 10% greater or lesser than absolute. All vertical directional terms, such as "up," "down," "above," "below," "vertical," or "height" used in the following description refer only to the orientation of features as depicted in the figure being described. Such directional terms are not intended to suggest that any features of the devices described herein must exist in any particular orientation when constructed.

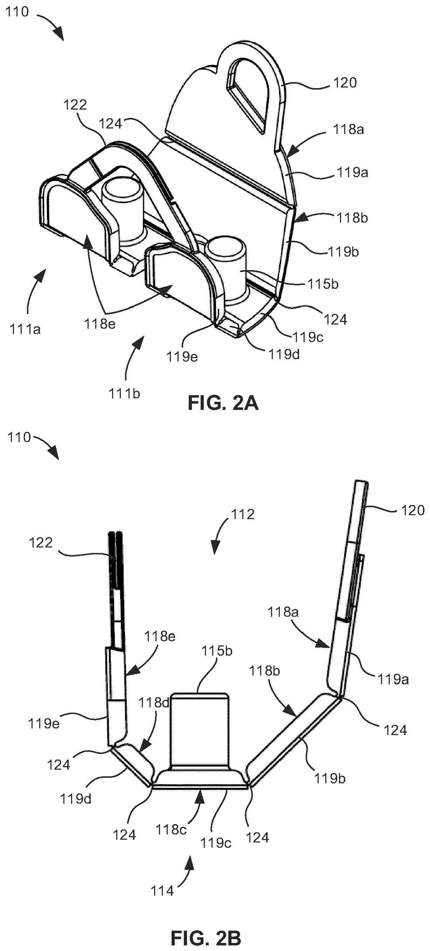

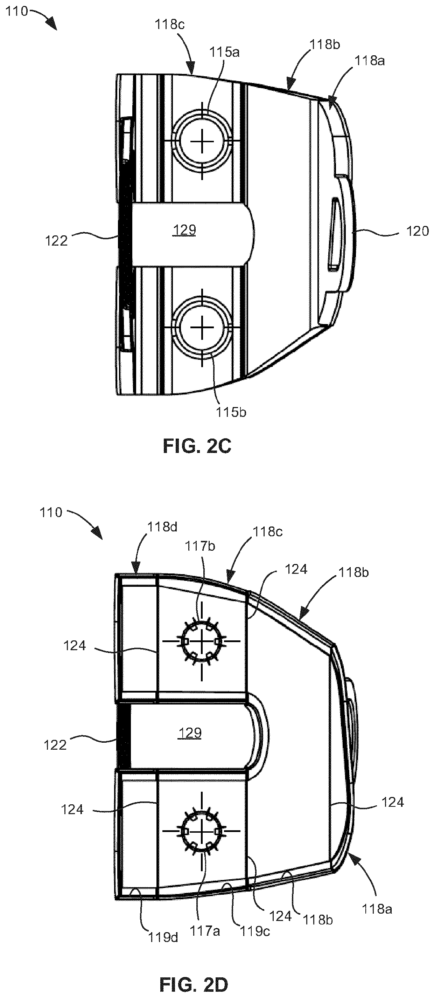

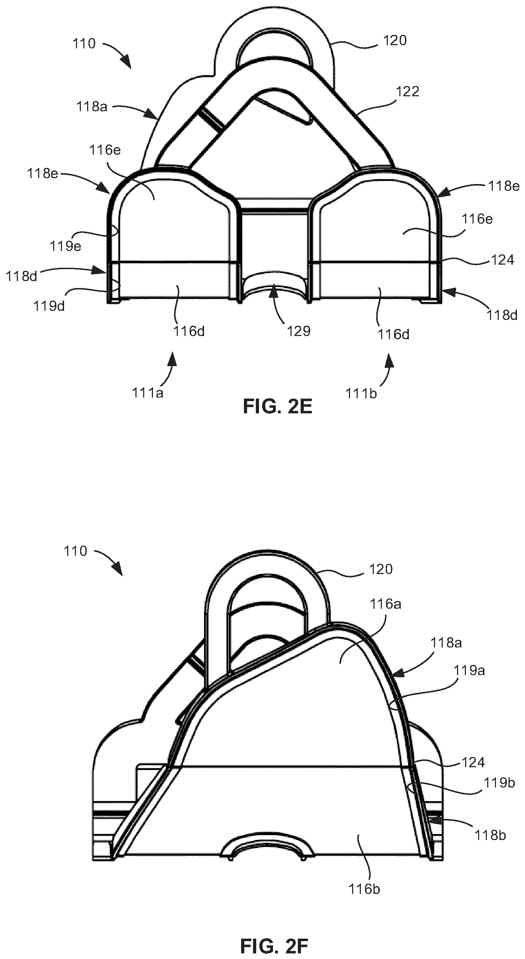

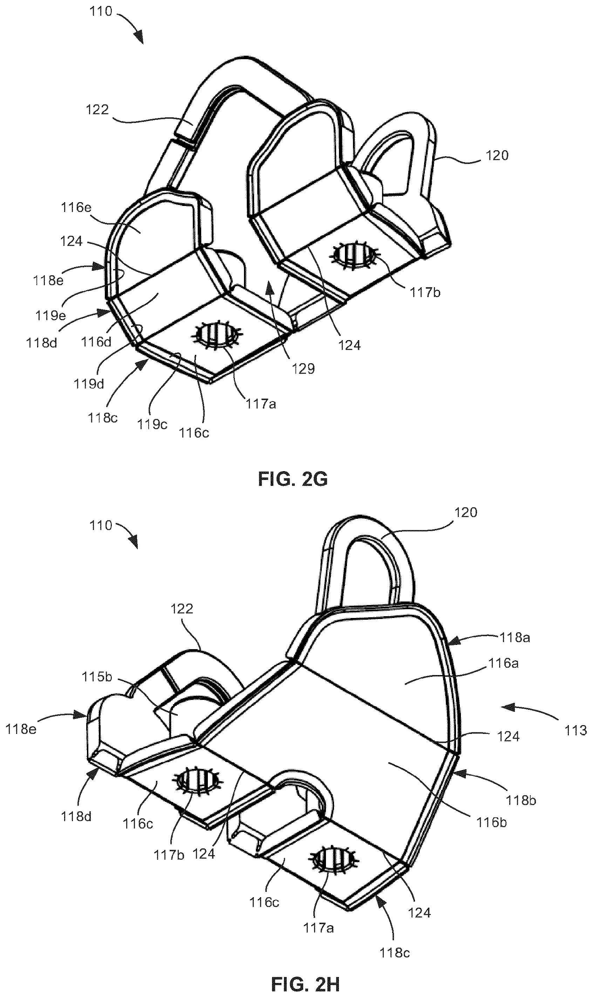

FIGs. 2A-2H depict a fixture 110 according to an embodiment of the present disclosure. Fixture 110 is configured to mask fixation side 102 of femoral component 100 during a coating process, such as PVD of TiN, on articular side 104 of femoral component 100.

Fixture or mask 110 generally includes a plurality of interconnected panels 118. In the particular embodiment depicted, fixture includes an anterior panel or first panel 118a, an anterior chamfer panel or second panel 118b, a distal panel or third panel 118c, a posterior chamfer panel or fourth panel 118d, and a posterior panel or fifth panel 118e. Anterior and anterior chamfer panels 118a-b generally form an anterior flange portion 113 configured to interface with an anterior flange 103 of femoral component 100. In this regard, anterior and anterior chamfer panels 118a-b are sized and shaped similarly to anterior surface 108a and anterior chamfer surface 108b of femoral component 100, respectively. Additionally, distal, posterior chamfer, and posterior panels 118c-e are intersected by a recess or notch 129 so as to form a first condylar portion 111a corresponding to first condyle 101a and a second condylar portion 111b corresponding to second condyle 101b. In this regard, distal, posterior chamfer, and posterior panels 118c-e are sized and shaped similarly to distal, posterior chamfer, and posterior surfaces 108c-e of femoral component 100, respectively. In other words, a perimeter of each panel 118a-e has a size and shape (i.e., peripheral shape) that is substantially the same as that of a corresponding femoral component bone interface surface 108a-e. Although fixture 110 is depicted as including five panels 118a-e, fixture may have more or less panels 118 depending on the construction of the femoral component it is configured to mask. For example, in some embodiments, femoral component 100 may have only three intersecting planar surfaces 108. In such embodiment, fixture 110 may then correspondingly have three interconnected panels 118-one for each surface. Fixture can also be provided in a multitude of different sizes each corresponding to a particular size of femoral component.

Fixture defines a mask side or first side 112 and an implant facing side or second side 114. Each panel 118a-e has an implant interface surface 116 at the implant facing side of fixture that is generally planar and configured to abut or otherwise rest upon a corresponding planar bone facing surface 108a-e of femoral component 100. In particular, anterior panel 118a includes an anterior interface surface or first planar surface 116a, anterior chamfer panel 118b includes an anterior chamfer interface surface or second planar surface 116b, distal panel 118c includes a distal interface surface or third planar surface 116c, posterior chamfer panel 118d includes a posterior chamfer interface surface or fourth planar surface 116c, and posterior panel 118e includes a posterior interface surface or fifth planar surface 116e. Some femoral components 100 may include a porous structure at bone facing side 102 of one or more bone interface surface 108. Other femoral components 100 may include depressions configured to receive bone cement. In both such porous and cemented femoral component embodiments, the bone facing surfaces 108 thereof still nonetheless form a generally planar surface even if each of such surface is partially or wholly constructed of a porous material or has depressions within it. In this regard, panels 118a-e are correspondingly constructed with planar interface surfaces 116a-e.

However, in other embodiments, fixation side 102 of femoral component 100 may also or exclusively include curved bone facing surfaces or may have planar surfaces or surface features differently arranged than what is depicted in FIGs. 1A-1C. For example, surfaces 108a-e may slope from a centerline of femoral component 100 to lateral and medial sides thereof. In such embodiments, each panel 118 of a plurality of panels may have corresponding surface features to receive or engage those of the femoral component in a conformal manner. For example, a raised surface feature extending from a fixation side 102 of a femoral component 100 may have a corresponding depression in a panel of fixture 110. Such exemplary femoral component bone facing/fixation side configurations for which fixture 110can be adapted can be found in U.S. Patent No. 10,219,908, which hereby incorporated by reference herein in its entirety.

Additionally, each panel 118a-e includes a sidewall 119a-e that extends along its perimeter and extends downwardly in a direction toward the implant facing side. More particular, first panel 118a includes a first sidewall, second panel includes a second sidewall, third panel includes a third sidewall, fourth panel includes a fourth sidewall, and fifth panel includes a fifth sidewall. Such sidewalls 119a-e together form a skirt 119 that traces a periphery of femoral component 100 so that, when fixture 110 is mounted onto femoral component 100, such skirt 119 overlaps a peripheral edge 100a of component 100 which helps seal bone facing side 102 from the infiltration of a coating material during a coating process.

Femoral component 100 may include one or more fixation member 105. In the embodiment depicted, femoral component 100 include a first peg or first fixation member 105a and a second peg or second fixation member 105b each extending from distal surface 108c. It may be desirable to also mask fixation members 105a-b from the coating material and to also ensure that fixture 110 is not impeded from properly seating onto femoral component 100 by fixation members 105a-b. In this regard, fixture 110 may include corresponding sheaths 115a-b to house fixation members 105a-b. In the particular embodiment depicted, distal panel 118c includes a first sheath or peg housing 115a and a second sheath or peg housing 115b. Each sheath 115a-b extends away from mask side 112 of distal panel 118c and may be cylindrical in shape, for example. At the implant facing side 114 of fixture 110, sheath openings 117a-b extend into sheaths 115a-b, respectively, which are each dimensioned to receive a corresponding fixation member 105a-b.

Each panel 118a-e is interconnected with an adjacent panel 118a-e via a hinge 124 allowing panels 118a-e to pivot relative to each adjacent panel 118a-e. For example, the hinge 124 connecting anterior panel 118a and anterior chamfer panel 118b allows anterior panel 118a and anterior chamfer panel 118b to pivot about a hinge axis relative to each other. In the embodiment depicted, hinges 124 are each a flexible hinge that may be integrally connected to panels 118a-e so that panels 118a-e and hinges 124 form a monolithic structure. In this regard, fixture 110 may be made from a polymer material, such as high-density polyethylene ("HDPE") or analogues thereof, for example, and injection molded or additively manufactured so that hinges 124 have a sufficient flexibility. In other embodiments, panels 118a-e may be formed separately and connected together via hinge pins, for example. In such embodiment, fixture 110 may be made from a metallic material or polymer material.

Fixture 110 also includes an anterior tab or first tab 120 and a posterior tab or second tab 122 to facilitate user manipulation. In this regard, anterior tab 120 is connected to and extends from sidewall 119a of anterior panel 118a, and posterior tab 122 is connected to and extends from sidewall 119e of posterior panel 118e. Also, posterior tab 122 extends from first condylar portion 111a to second condylar portion 111b across recess 129 which helps provide rigidity to the posterior end of fixture 110.

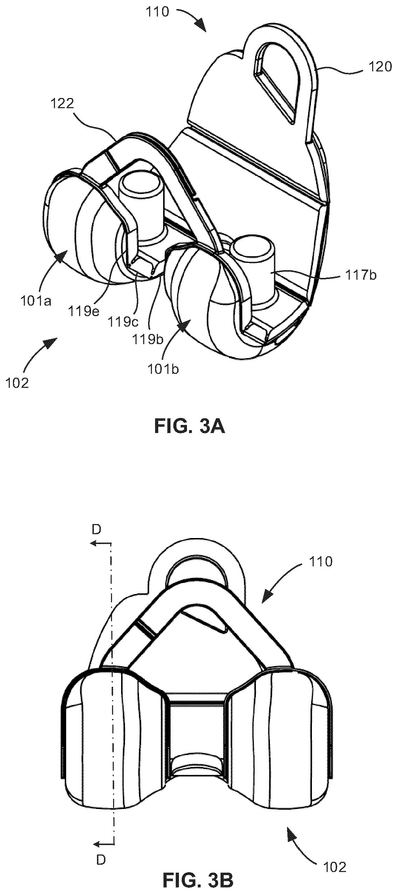

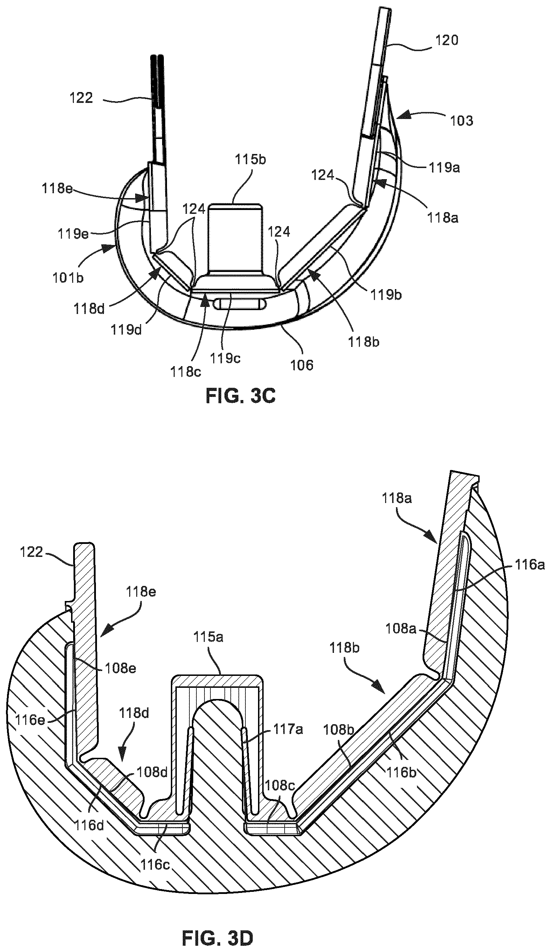

FIGs. 3A-3D depict fixture 110 coupled with femoral component 100. In this regard, prior to subjecting femoral component 100 to a surface coating process, fixture 110 is mounted onto bone facing side 102 thereof by gripping tabs 120, 122 and moving tabs 120, 122 toward each other. This causes panels 118a-e to fold inwardly toward sheaths 115a-b allowing fixture 110 to easily fit within bone facing side 102 of femoral component 100. Fixture 110 is then mounted onto femoral component 100 so that distal panel 118c is seated on distal surface 108c and fixation members 105a-b are received in sheaths 115a-b, as best shown in the cross-section of FIG. 3D. Thereafter, tabs 120, 122 are moved apart unfolding fixture 110 and so that the remaining panels 118a, 118b, 118d, 118e engage their respective surfaces 108a, 108b, 108d, 108e. Skirt 119 of fixture 110 extends over the peripheral edge 100a of femoral component 100, as best shown in FIGs. 3A and 3C. Once the coating process is completed, tabs 120, 122 are moved back toward each other, and fixture 110 is lifted away from bone contacting side 102 of femoral component 100. This can be done without touching the articular side 104 of femoral component 100.

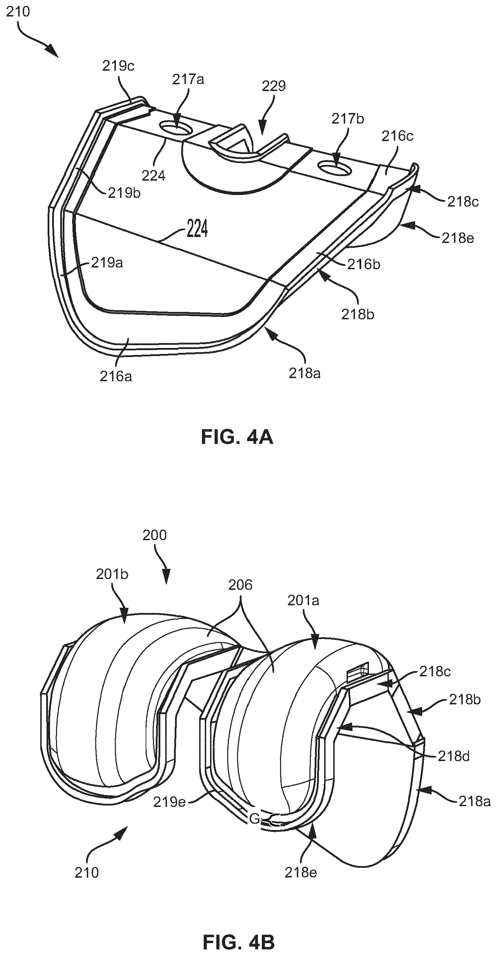

FIGs. 4A and 4B depict a fixture 210 according to another embodiment of the present disclosure. For ease of review, like elements will be accorded like reference numerals to that of fixture 110, but within the 200-series of numbers. For instance, fixture 210 includes a plurality of panels 218a-e each configured to correspond to a respective surface 108a-e of femoral component 100. However, fixture 210 also differs from fixture 110 in that panels 218a-e are rigidly fixed relative to each other. However, in some embodiments, panels 218a-e may be hingedly connected, as discussed above. Additionally, while panels 218a-e together have the same general shape as that of femoral component 100, panels 218a-e are dimensioned to be larger than femoral component 100 so that, when fixture 210 is mounted to femoral component 100, a gap G is formed between the peripheral edge 100a of femoral component 100 and skirt 219, as best shown in FIG. 4B. Such gap G may be about 1 to 2 mm.

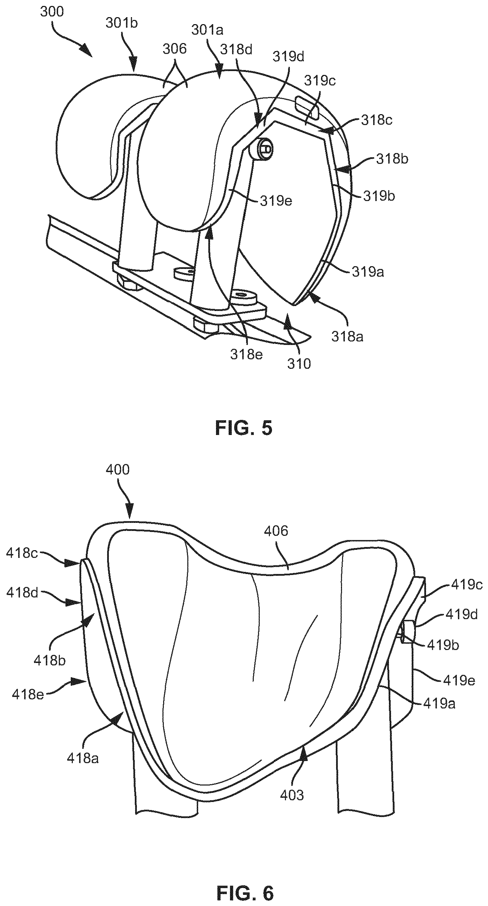

FIG. 5 depicts a fixture 310 according to a further embodiment of the present disclosure. For ease of review, like elements will be accorded like reference numerals to that of fixture 110, but within the 300-series of numbers. For instance, fixture 310 includes a plurality of panels 318a-e each configured to correspond to a respective surface 108a-e of femoral component 100. However, fixture 310 differs from fixture 110 in that sidewalls 219a-e do not extend downwardly and instead terminate so that they are flush with the peripheral edge 100a of femoral component 100 when mounted thereto.

FIG. 6 depicts a fixture 410 according to yet another embodiment of the present disclosure. For ease of review, like elements will be accorded like reference numerals to that of fixture 110, but within the 400-series of numbers. For instance, fixture 410 includes a plurality of panels 418a-e each configured to correspond to a respective bone contact surface 108a-e of femoral component 100. However, fixture 410 differs from fixture 110 in that sidewalls 419a-e do not extend downwardly and instead extend outwardly so that they project from the peripheral edge 100a of femoral component 100 when fixture 410 is mounted thereto. In other words, panels 418a-e together define a perimeter greater than that of femoral component 100.

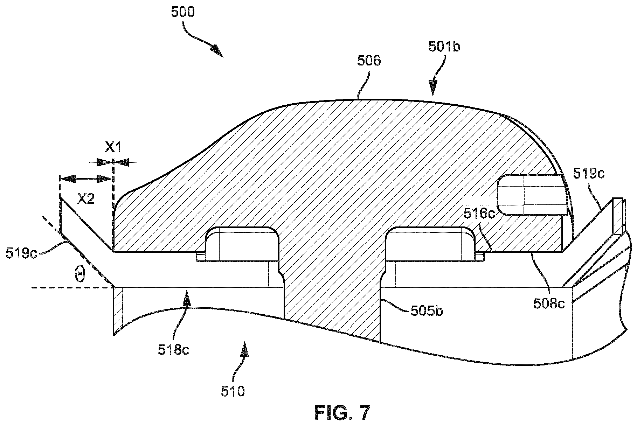

FIG. 7 depicts a fixture 510 according to an even further embodiment of the present disclosure. For ease of review, like elements will be accorded like reference numerals to that of fixture 110, but within the 500-series of numbers. For instance, fixture 510 includes a plurality of panels 518a-e each configured to correspond to a respective surface 108a-e of femoral component 100. However, fixture 510 differs from fixture 110 in that sidewalls 519a-e are not perpendicular to implant interface surfaces 516a-e. Instead, sidewalls 519a-e extend at a downwardly depending oblique angle Θ relative to their respective bone interface surface 516a-e. Such angle Θ may be about 45 degrees, for example. Additionally, sidewalls 519a-e are offset from a peripheral edge 100a of femoral component 100 by a first dimension X1 and extends to a free end an additional distance X2. X1 may be about 0.5 mm, and X2 may be about 3.5 mm, for example. Such angled sidewalls 519a-e create clearance for coating material to coat side edges of femoral component 100 while continuing to mask bone facing side 102 thereof.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A fixture (110, 210, 310, 410, 510) configured for masking a femoral component (100) during a surface coating process, the femoral component comprising an articular side (104) , a bone facing side (102), and a peripheral edge (100a) defining an intersection between the articular side (104) and bone facing side (102), the bone facing side having a first planar surface (116a) having a first perimeter and a second planar surface (116b) having a second perimeter, the perimeters of the first and second planar surfaces (116a, 116b) defining at least a portion of the peripheral edge (100a) of the femoral component (100), the fixture (110, 210, 310, 410, 510) comprising:
a first panel (118a, 218a, 318a, 418a, 518a) having a first interface surface defining a first perimeter of the first panel (118a, 218a, 318a, 418a, 518a), the first perimeter of the first panel (118a, 218a, 318a, 418a, 518a) having substantially the same shape as the first perimeter of the first planar surface (116a) of the femoral component (100); and
a second panel (118b, 218b, 318b, 418b, 518b) connected to the first panel (118a, 218a, 318a, 418a, 518a) and having a second interface surface defining a second perimeter of the second panel (118b, 218b, 318b, 418b, 518b), the second perimeter of the second panel (118b, 218b, 318b, 418b, 518b) having substantially the same shape as the second perimeter of the second planar surface (116b) of the femoral component (100),
wherein, when the fixture (110, 210, 310, 410, 510) is mounted to the bone facing side (102) of the femoral component (100), the first interface surface is configured to engage the first planar surface (116a) of the femoral component (100) and cover an entirety thereof, and the second interface surface is configured to engage the second planar surface (116b) of the femoral component (100) and cover an entirety thereof.

2. The fixture (110, 210, 510) of claim 1, wherein the first panel (118a, 218a, 518a) includes a first sidewall (119a, 219a, 519a) extending downwardly from the first perimeter, and the second panel (118b, 218b, 518b) includes a second sidewall (119b, 219b, 519b) extending downwardly from the second perimeter, the first and second sidewalls being configured to overlap at least a portion of the peripheral edge (100a) of the femoral component (100) when the fixture (110, 210, 510) is mounted to the bone facing side (102) of the femoral component (100).

3. The fixture (110, 210) of claim 2, wherein the first and second sidewalls (119a, 119b, 219a, 219b) are perpendicular to the first and second interface surfaces of the first and second panels (118a, 118b, 218a, 218b,), respectively.

4. The fixture (510) of claim 2, wherein the first and second sidewalls (519a, 519b) intersect the first and second interface surfaces, respectively, at an oblique angle.

5. The fixture (510) of claim 4, wherein the oblique angle is 45 degrees.

6. The fixture (510) of any one of claims 2-5, wherein the first and second sidewalls (519a, 519b) of the respective first and second panels (518a, 518b) are each offset from the peripheral edge (100a) of the femoral component (100) by a distance.

7. The fixture (510) of claim 6, wherein the distance is 0.5 mm.

8. The fixture (210) of any one of claims 1-7, wherein the first and second panels (218a, 218b) are rigidly fixed to each other.

9. The fixture (110, 210) of any one of claims 1-7, wherein the first and second panels (118a, 118b, 218a, 218b) are pivotable relative to each other via a hinge (124, 224).

10. The fixture (110, 210) of claim 9, wherein the hinge (124, 224) is a flexible hinge and the flexible hinge (124, 224) and first and second panels (118a, 118b, 218a, 218b) are integrally connected so as to form a monolithic structure.

11. The fixture (110, 210, 310, 410, 510) of any one of claims 1-10, further comprising a third panel (118c, 218c, 318c, 418c, 518c) connected to the second panel (118b, 218c, 318c, 418c, 518c) and having a third interface surface defining a third perimeter of the third panel (118c, 218c, 318c, 418c, 518c), the third perimeter having substantially the same shape as a third perimeter of a third planar surface (116c) of the femoral component (100).

12. The fixture (110, 210, 310, 410, 510) of claim 11, wherein the third panel (118c, 218c, 318c, 418c, 518c) includes first and second sheaths (115a, 115b, 215a, 215b, 315a, 315b, 415a, 415b, 515a, 515b) extending from a mask side (112, 212, 312, 412, 512) of the fixture (110, 210, 310, 410, 510) and first and second sheath openings (117a, 117b, 217a, 217b, 317a, 317b, 417a, 417b, 517a, 517b) extending into the respective first and second sheaths (115a, 115b, 215a, 215b, 315a, 315b, 415a, 415b, 515a, 515b) from an implant facing side (114) of the fixture (110), the first and second sheath openings (117a, 117b, 217a, 217b, 317a, 317b, 417a, 417b, 517a, 517b) being configured to respectively receive first and second fixation members (105a, 105b, 205a, 205b, 305a, 305b, 405a, 405b, 505a, 505b) extending from the third planar surface (116c) of the femoral component (100) when the fixture (110, 210, 310, 410, 510) is mounted to the bone facing side (102) of the femoral component (100).

13. The fixture (110, 210, 310, 410, 510) of any one of claims 11-12, further comprising a fourth and fifth panel having (118d, 118e, 218d, 218e, 318d, 318e, 418d, 418e, 518d, 518e) respective fourth and fifth interface surfaces corresponding to respective fourth and fifth planar surfaces (116d, 116e) of the femoral component (100), wherein a recess (129, 229, 329, 429, 529) intersects the third, fourth, and fifth panels (118c, 118d, 118e, 218c, 218d, 218e, 318c, 318d, 318e, 418c, 418d, 418e, 518c, 518d, 518e) of the fixture (110, 210, 310, 410, 510) so as to define first and second condylar portions (111a, 111b, 211a, 211b, 311a, 311b, 411a, 411b, 511a, 511b) of the fixture (110, 210, 310, 410, 510).

14. The fixture (110, 210, 310, 410, 510) of claim 13, wherein the fixture (110, 210, 310, 410, 510) further includes a first tab (120, 220, 320, 420, 520) extending from the first panel (118a, 218a, 318a, 418a, 518a), and a second tab (122, 222, 322, 422, 522) extending from the fifth panel (118e, 218e, 318e, 418e, 518e) across the recess.

15. The fixture (310) of any one of claims 1-14, wherein the first and second perimeters of the first and second panels (318a, 318b) are configured to be flush with the first and second perimeters of the first and second planar surfaces (316a, 316b) of the femoral component (100) when the fixture (110) is mounted to the bone facing side (102) of the femoral component (100).

## Patentansprüche

1. Befestigung (110, 210, 310, 410, 510), die zum Maskieren einer Femurkomponente (100) während eines Oberflächenbeschichtungsprozesses ausgestaltet ist, wobei die Femurkomponente eine Gelenkseite (104), eine dem Knochen zugewandte Seite (102) und einen umlaufenden Rand (100a) umfasst, der eine Schnittstelle zwischen der Gelenkseite (104) und der dem Knochen zugewandten Seite (102) definiert, wobei die dem Knochen zugewandte Seite eine erste ebene Oberfläche (116a), die einen ersten Umkreis aufweist, und eine zweite ebene Oberfläche (116b) aufweist, die einen zweiten Umkreis aufweist, wobei die Umkreise der ersten und der zweiten ebenen Oberfläche (116a, 116b) mindestens einen Abschnitt des umlaufenden Randes (100a) der Femurkomponente (100) definieren, wobei die Befestigung (110, 210, 310, 410, 510) umfasst:
eine erste Platte (118a, 218a, 318a, 418a, 518a), die eine ersten Grenzoberfläche aufweist, die einen ersten Umkreis der ersten Platte (118a, 218a, 318a, 418a, 518a) definiert, wobei der erste Umkreis der ersten Platte (118a, 218a, 318a, 418a, 518a) im Wesentlichen die gleiche Form wie der erste Umkreis der ersten ebenen Oberfläche (116a) der Femurkomponente (100) aufweist; und
eine zweite Platte (118b, 218b, 318b, 418b, 518b), die mit der ersten Platte (118a, 218a, 318a, 418a, 518a) verbunden ist und eine zweite Grenzoberfläche aufweist, die einen zweiten Umkreis der zweiten Platte (118b, 218b, 318b, 418b, 518b) definiert, wobei der zweite Umkreis der zweiten Platte (118b, 218b, 318b, 418b, 518b) im Wesentlichen die gleiche Form wie der zweite Umkreis der zweiten ebenen Oberfläche (116b) der Femurkomponente (100) aufweist,
wobei, wenn die Befestigung (110, 210, 310, 410, 510) an der dem Knochen zugewandten Seite (102) der Femurkomponente (100) montiert ist, die erste Grenzoberfläche dazu ausgestaltet ist, in die erste ebene Oberfläche (116a) der Femurkomponente (100) einzugreifen und eine Gesamtheit davon zu bedecken, und die zweite Grenzoberfläche dazu ausgestaltet ist, in die zweite ebene Oberfläche (116b) der Femurkomponente (100) einzugreifen und eine Gesamtheit davon zu bedecken.

2. Befestigung (110, 210, 510) nach Anspruch 1, wobei die erste Platte (118a, 218a, 518a) eine erste Seitenwand (119a, 219a, 519a) umfasst, die sich vom ersten Umkreis abwärts erstreckt, und die zweite Platte (118b, 218b, 518b) eine zweite Seitenwand (119b, 219b, 519b) umfasst, die sich von dem zweiten Umkreis abwärts erstreckt, wobei die erste und die zweite Seitenwand dazu ausgestaltet sind, zumindest einen Abschnitt des umlaufenden Randes (110a) der Femurkomponente (100) zu überlappen, wenn die Befestigung (110, 210, 510) an der dem Knochen zugewandten Seite (102) der Femurkomponente (100) montiert ist.

3. Befestigung (110, 210) nach Anspruch 2, wobei die erste und die zweite Seitenwand (119a 119b, 219a, 219b) senkrecht zur ersten und zur zweiten Grenzoberfläche der ersten beziehungsweise zweiten Platte (118a, 118b, 218a, 218b) sind.

4. Befestigung (510) nach Anspruch 2, wobei die erste und die zweite Seitenwand (519a, 519b) die erste beziehungsweise die zweite Grenzoberfläche in einem schiefen Winkel schneiden.

5. Befestigung (510) nach Anspruch 4, wobei der schiefe Winkel 45 Grad beträgt.

6. Befestigung (510) nach einem der Ansprüche 2 bis 5, wobei die erste und die zweite Seitenwand (519a, 519b) der ersten beziehungsweise der zweiten Platte (518a, 518b) jeweils von dem umlaufenden Rand (100a) der Femurkomponente (100) um einen Abstand versetzt sind.

7. Befestigung (510) nach Anspruch 6, wobei der Abstand 0,5 mm beträgt.

8. Befestigung (210) nach einem der Ansprüche 1 bis 7, wobei die erste und die zweite Platte (218a, 218b) starr aneinander befestigt sind.

9. Befestigung (110, 210) nach einem der Ansprüche 1 bis 7, wobei die erste und die zweite Platte (118a, 118b, 218a, 218b) über ein Scharnier (124, 224) in Bezug aufeinander schwenkbar sind.

10. Befestigung (110, 210) nach Anspruch 9, wobei das Scharnier (124, 224) ein flexibles Scharnier ist und das flexible Scharnier (124, 224) und die erste und die zweite Platte (118a, 118b, 218a, 218b) einteilig verbunden sind, um eine monolithische Struktur zu bilden.

11. Befestigung (110, 210, 310, 410, 510) nach einem der Ansprüche 1 bis 10, ferner umfassend eine dritte Platte (118c, 218c, 318c, 418c, 518c), die mit der zweiten Platte (118b, 218c, 318c, 418c, 518c) verbunden ist und eine dritte Grenzoberfläche aufweist, die einen dritten Umkreis der dritten Platte (118c, 218c, 318c, 418c, 518c) bildet, wobei der dritte Umkreis im Wesentlichen die gleiche Form wie ein dritter Umkreis einer dritten ebenen Oberfläche (116c) der Femurkomponente (100) aufweist.

12. Befestigung (110, 210, 310, 410, 510) nach Anspruch 11, wobei die dritte Platte (118c, 218c, 318c, 418c, 518c) eine erste und eine zweite Hülle (115a, 115b, 215a, 215b, 315a, 315b, 415a, 415b, 515a, 515b), die sich von einer Maskenseite (112, 212, 312, 412, 512) der Befestigung (110, 210, 310, 410, 510) erstrecken, und eine erste und eine zweite Hüllenöffnung (117a, 117b, 217a, 217b, 317a, 317b, 417a, 417b, 517a, 517b) umfasst, die sich von einer einem Implantat zugwandten Seite (114) der Befestigung (110) in die erste beziehungsweise zweite Hülle (115a, 115b, 215a, 215b, 315a, 315b, 415a, 415b, 515a, 515b) erstrecken, wobei die erste und die zweite Hüllenöffnung (117a, 117b, 217a, 217b, 317a, 317b, 417a, 417b, 517a, 517b) dazu ausgestaltet sind, ein erstes beziehungsweise ein zweites Befestigungselement (105a, 105b, 205a, 205b, 305a, 305b, 405a, 405b, 505a, 505b) aufzunehmen, die sich von der dritten ebenen Oberfläche (116c) der Femurkomponente (110) erstrecken, wenn die Befestigung (110, 210, 310, 410, 510) an der dem Knochen zugewandten Seite (102) der Femurkomponente (100) montiert ist.

13. Befestigung (110, 210, 310, 410, 510) nach einem der Ansprüche 11 bis 12, ferner umfassend eine vierte und eine fünfte Platte (118d, 118e, 218d, 218e, 318d, 318e, 418d, 418e, 518d, 518e), die eine vierte beziehungsweise fünfte Grenzoberfläche aufweisen, die einer vierten beziehungsweise fünften ebenen Oberfläche (116d, 116e) der Femurkomponente (100) entsprechen, wobei eine Vertiefung (129, 229, 329, 429, 529) die dritte, vierte und fünfte Platte (118c, 118d, 118e, 218c, 218d, 218e, 318c, 318d, 318e, 418c, 418d, 418e, 518c, 518d, 518e) der Befestigung (110, 210, 310, 410, 510) schneidet, um einen ersten und einen zweiten Kondylenabschnitt (111a, 111b, 211a, 211b, 311a, 311b, 411a, 411b, 511a, 511b) der Befestigung (110, 210, 310, 410, 510) zu definieren.

14. Befestigung (110, 210, 310, 410, 510) nach Anspruch 13, wobei die Befestigung (110, 210, 310, 410, 510) ferner eine erste Lasche (120, 220, 320, 420, 520), die sich von der ersten Platte (118a, 218a, 318a, 418a, 518a) erstreckt, und eine zweite Lasche (122, 222, 322, 422, 522) umfasst, die sich von der fünften Platte (118e, 218e, 318e, 418e, 518e) durch die Vertiefung erstreckt.

15. Befestigung (310) nach einem der Ansprüche 1 bis 14, wobei der erste und der zweite Umkreis der ersten und der zweiten Platte (318a, 318b) dazu ausgestaltet sind, mit dem ersten und dem zweiten Umkreis der ersten und der zweiten ebenen Oberfläche (316a, 316b) der Femurkomponente (100) bündig zu sein, wenn die Befestigung (110) an der dem Knochen zugewandten Seite (102) der Femurkomponente (100) montiert ist.

## Revendications

1. Fixation (110, 210, 310, 410, 510) configurée pour masquer un composant fémoral (100) pendant un processus de revêtement de surface, le composant fémoral comprenant un côté articulaire (104), un côté face à l'os (102), et un bord périphérique (100a) définissant une intersection entre le côté articulaire (104) et le côté face à l'os (102), la face tournée vers l'os ayant une première surface plane (116a) ayant un premier périmètre et une deuxième surface plane (116b) ayant un deuxième périmètre, les périmètres des première et deuxième surfaces planes (116a, 116b) définissant au moins une partie du bord périphérique (100a) du composant fémoral (100), la fixation (110, 210, 310, 410, 510) comprenant :
un premier panneau (118a, 218a, 318a, 418a, 518a) ayant une première surface d'interface définissant un premier périmètre du premier panneau (118a, 218a, 318a, 418a, 518a), le premier périmètre du premier panneau (118a, 218a, 318a, 418a, 518a) ayant sensiblement la même forme que le premier périmètre de la première surface plane (116a) du composant fémoral (100) ; et
un deuxième panneau (118b, 218b, 318b, 418b, 518b) relié au premier panneau (118a, 218a, 318a, 418a, 518a) et présentant une deuxième surface d'interface définissant un deuxième périmètre du deuxième panneau (118b, 218b, 318b, 418b, 518b), le deuxième périmètre du deuxième panneau (118b, 218b, 318b, 418b, 518b) ayant sensiblement la même forme que le deuxième périmètre de la deuxième surface plane (116b) du composant fémoral (100),
dans laquelle, lorsque la fixation (110, 210, 310, 410, 510) est montée sur le côté face à l'os (102) du composant fémoral (100), la première surface d'interface est configurée pour s'engager dans la première surface plane (116a) du composant fémoral (100) et en recouvrir la totalité, et la deuxième surface d'interface est configurée pour s'engager dans la deuxième surface plane (116b) du composant fémoral (100) et en recouvrir la totalité.

2. Fixation (110, 210, 510) selon la revendication 1, dans laquelle le premier panneau (118a, 218a, 518a) comprend une première paroi latérale (119a, 219a, 519a) s'étendant vers le bas à partir du premier périmètre, et le deuxième panneau (118b, 218b, 518b) comprend une deuxième paroi latérale (119b, 219b, 519b) s'étendant vers le bas à partir du deuxième périmètre, les première et deuxième parois latérales étant configurées pour chevaucher au moins une partie du bord périphérique (100a) du composant fémoral (100) lorsque la fixation (110, 210, 510) est montée sur le côté face à l'os (102) du composant fémoral (100).

3. Fixation (110, 210) selon la revendication 2, dans laquelle les première et deuxième parois latérales (119a, 119b, 219a, 219b) sont perpendiculaires aux première et deuxième surfaces d'interface des premier et deuxième panneaux (118a, 118b, 218a, 218b,), respectivement.

4. Fixation (510) selon la revendication 2, dans laquelle les première et deuxième parois latérales (519a, 519b) coupent les première et deuxième surfaces d'interface, respectivement, à un angle oblique.

5. Fixation (510) selon la revendication 4, dans laquelle l'angle oblique est de 45 degrés.

6. Fixation (510) selon l'une quelconque des revendications 2 à 5, dans laquelle les première et deuxième parois latérales (519a, 519b) des premier et deuxième panneaux respectifs (518a, 518b) sont chacune décalées d'une certaine distance par rapport au bord périphérique (100a) du composant fémoral (100).

7. Fixation (510) selon la revendication 6, dans laquelle la distance est de 0,5 mm.

8. Fixation (210) selon l'une quelconque des revendications 1 à 7, dans laquelle les premier et deuxième panneaux (218a, 218b) sont fixés de manière rigide l'un à l'autre.

9. Fixation (110, 210) selon l'une quelconque des revendications 1 à 7, dans laquelle les premier et deuxième panneaux (118a, 118b, 218a, 218b) peuvent pivoter l'un par rapport à l'autre par l'intermédiaire d'une charnière (124, 224).

10. Fixation (110, 210) selon la revendication 9, dans laquelle la charnière (124, 224) est une charnière flexible et la charnière flexible (124, 224) et les premier et deuxième panneaux (118a, 118b, 218a, 218b) sont intégralement reliés de manière à former une structure monolithique.

11. Fixation (110, 210, 310, 410, 510) selon l'une quelconque des revendications 1 à 10, comprenant en outre un troisième panneau (118c, 218c, 318c, 418c, 518c) relié au deuxième panneau (118b, 218c, 318c, 418c, 518c) et présentant une troisième surface d'interface définissant un troisième périmètre du troisième panneau (118c, 218c, 318c, 418c, 518c), le troisième périmètre ayant sensiblement la même forme qu'un troisième périmètre d'une troisième surface plane (116c) du composant fémoral (100).

12. Fixation (110, 210, 310, 410, 510) selon la revendication 11, dans laquelle le troisième panneau (118c, 218c, 318c, 418c, 518c) comprend des première et deuxième gaines (115a, 115b, 215a, 215b, 315a, 315b, 415a, 415b, 515a, 515b) s'étendant à partir d'un côté du masque (112, 212, 312, 412, 512) du dispositif (110, 210, 310, 410, 510) et les première et deuxième ouvertures de gaine (117a, 117b, 217a, 217b, 317a, 317b, 417a, 417b, 517a, 517b) s'étendant dans les première et deuxième gaines respectives (115a, 115b, 215a, 215b, 315a, 315b, 415a, 415b, 515a, 515b) à partir d'un côté faisant face à l'implant (114) de la fixation (110), les première et deuxième ouvertures de gaine (117a, 117b, 217a, 217b, 317a, 317b, 417a, 417b, 517a, 517b) étant configurées pour recevoir respectivement les premier et deuxième éléments de fixation (105a, 105b, 205a, 205b, 305a, 305b, 405a, 405b, 505a, 505b) s'étendant à partir de la troisième surface plane (116c) du composant fémoral (100) lorsque la fixation (110, 210, 310, 410, 510) est montée sur le côté face à l'os (102) du composant fémoral (100).

13. Fixation (110, 210, 310, 410, 510) selon l'une quelconque des revendications 11 à 12, comprenant en outre un quatrième et un cinquième panneau ayant (118d, 118e, 218d, 218e, 318d, 318e, 418d, 418e, 518d, 518e) des quatrième et cinquième surfaces d'interface respectives correspondant aux quatrième et cinquième surfaces planes respectives (116d, 116e) du composant fémoral (100), dans lequel un renfoncement (129, 229, 329, 429, 529) coupe les troisième, quatrième et cinquième panneaux (118c, 118d, 118e, 218c, 218d, 218e, 318c, 318d, 318e, 418c, 418d, 418e, 518c, 518d, 518e) de la fixation (110, 210, 310, 410, 510) de manière à définir les première et deuxième parties condylaires (111a, 111b, 211a, 211b, 311a, 311b, 411a, 411b, 511a, 511b) de l'élément de fixation (110, 210, 310, 410, 510).

14. Fixation (110, 210, 310, 410, 510) selon la revendication 13, dans laquelle l'appareil (110, 210, 310, 410, 510) comprend en outre une première languette (120, 220, 320, 420, 520) s'étendant à partir du premier panneau (118a, 218a, 318a, 418a, 518a), et une deuxième languette (122, 222, 322, 422, 522) s'étendant à partir du cinquième panneau (118e, 218e, 318e, 418e, 518e) à travers le renfoncement.

15. Fixation (310) selon l'une quelconque des revendications 1 à 14, dans laquelle les premier et deuxième périmètres des premier et deuxième panneaux (318a, 318b) sont configurés pour affleurer les premier et deuxième périmètres des première et deuxième surfaces planes (316a, 316b) du composant fémoral (100) lorsque le dispositif (110) est monté sur le côté face à l'os (102) du composant fémoral (100).
